# FASCICULE DE BREVET EUROPEEN

(11) **EP 3 136 977 B1**
(45) Date de publication et mention de la délivrance du brevet: **28.03.2018**
(21) Numéro de dépôt: 15721648.2
(22) Date de dépôt: 30.04.2015
(51) Int. Cl.: A61B 10/02

(54) **DISPOSITIF DE PRELEVEMENT IN VIVO D'ESPECES BIOLOGIQUES**
VORRICHTUNG FÜR IN VIVO ENTNAHME VON BIOLOGISCHEN PROBEN
DEVICE FOR IN VIVO SAMPLING OF BIOLOGICAL SAMPLES

(30) Priorité: 30.04.2014 FR 1453945
(43) Date de publication de la demande: 08.03.2017
(73) Titulaire: Commissariat à l'Énergie Atomique et aux Énergies Alternatives, 75015 Paris (FR)
(72) Inventeur: MOMBRUN, Adrien, 38000 Grenoble (FR); BOUAMRANI, Mohamed-Ali, F-38000 Grenoble (FR); BOYER, Eric, F-38240 Meylan (FR); YEROMONAHOS, Christelle, F-69006 Lyon (FR)
(74) Mandataire: Regimbeau
(86) Numéro de dépôt international: PCT/EP2015/059450
(87) Numéro de publication internationale: WO 2015/166019

(56) Documents cités:
- EP-A1- 1 234 543
- EP-A1- 2 702 949
- US-A- 4 766 907
- US-A1- 2013 296 738

## Description

### DOMAINE DE L'INVENTION

La présente invention concerne un dispositif de prélèvement in vivo d'espèces biologiques.

### ARRIERE PLAN DE L'INVENTION

Des mécanismes cellulaires et moléculaires ont été mis en évidence dans l'altération de la fonction auditive, notamment dans la presbyacousie et le traumatisme sonore.

Il semble en particulier que les protéines contenues dans la périlymphe affectent le métabolisme et la distribution des médicaments délivrés à la cochlée. De nombreuses protéines semblent en effet se fixer aux médicaments libérés, les rendant inactifs contre les tissus ciblés [1].

Une analyse de la périlymphe a été réalisée chez des patients présentant un schwanome vestibulaire, fournissant un aperçu des protéines contenues dans la périlymphe [2]. Cette étude a mis en évidence la présence de deux protéines dans la périlymphe des patients atteints d'un schwanome vestibulaire. Ses auteurs insistent sur l'intérêt d'un recueil de périlymphe sans perte de matériel pour l'analyse du protéome et rapportent l'existence d'une contamination de l'échantillon recueilli par des éléments du sang (hémoglobine, kératine).

Dans la littérature, des analyses de la périlymphe obtenue par des prélèvements externes ont été décrites. Cependant, ces prélèvements sont soumis à une contamination par le liquide céphalo-rachidien ou font appel à des méthodes très complexes.

Ainsi, une étude réalisée sur le cochon d'Inde montre que la procédure d'aspiration d'échantillons au niveau du tour basal de la cochlée de la scala tympani a une influence importante sur la composition de la périlymphe [3]. Bien que la partie aspirée initialement semble contenir de la périlymphe pure, l'échantillon se contamine avec l'aspiration du liquide céphalo-rachidien. Les prélèvements réalisés au niveau du tour basal de la cochlée sont donc en réalité un mélange de périlymphe et de liquide céphalo-rachidien.

Les auteurs de l'étude [3] ont développé une nouvelle méthode pour obtenir des échantillons de périlymphe plus importants en la collectant sans aucune perte au niveau de l'apex cochléaire [4]. Néanmoins, les biais de prélèvement réalisés au niveau du tour basal de la cochlée restent une source majeure d'erreur de la compréhension de la pharmacocinétique de la périlymphe. D'autre part, le protocole de prélèvement selon cette méthode est très lourd et non réalisable en pratique in vivo.

Une méthodologie de prélèvement standardisée est donc nécessaire pour recueillir un fluide corporel présent dans une cavité, en particulier de la périlymphe lorsque la cavité est la fenêtre ronde de la cochlée. La demande de brevet européenne EP 2702949 divulgue un dispositif de prélèvement comprenant une couche polymérique poreuse.

### BREVE DESCRIPTION DE L'INVENTION

Un but de l'invention est de concevoir un dispositif de prélèvement d'espèces biologiques dans la cochlée ou d'autres glandes de petites dimensions, qui minimise voire évite toute contamination des espèces biologiques au cours du prélèvement.

Conformément à l'invention, il est proposé un dispositif de prélèvement in vivo d'espèces biologiques comprenant :
- une gaine tubulaire s'étendant entre une extrémité proximale de ladite gaine et une extrémité distale de ladite gaine, ladite extrémité distale de la gaine présentant une partie saillante adaptée pour perforer une membrane d'un organe contenant les espèces biologiques à prélever,
- une tige s'étendant entre une extrémité proximale de ladite tige et une extrémité distale de ladite tige, apte à coulisser dans la gaine entre une position rétractée dans laquelle l'extrémité distale de la tige est située à l'intérieur de la gaine et une position déployée dans laquelle l'extrémité distale de la tige s'étend au-delà de l'extrémité distale de la gaine,
ladite tige comprenant un support de capture desdites espèces biologiques, en un matériau poreux, agencé dans une région distale de la tige sur une portion de la circonférence de la tige de telle sorte que le support de capture soit situé à l'extérieur de la gaine lorsque la tige est dans sa position déployée.

Les termes « proximal » et « distal » sont définis dans le présent texte par rapport au praticien qui manipule le dispositif.

Par « poreux », on entend dans le présent texte une densité de pores comprise entre 10 et 75% et une taille des pores comprise entre 1 et 100 nm.

Par espèces biologiques, on entend des protéines, peptides, métabolites, ou toute molécule organique susceptible d'être présente dans un organisme. Il peut également s'agir de cellules, bactéries, virus, ou autres microorganismes.

Selon un mode de réalisation, l'extrémité distale de la gaine tubulaire forme un biseau.

De manière particulièrement avantageuse, l'extrémité distale de la tige comprend un embout arrondi.

Selon un mode de réalisation, ledit embout est réalisé en un polymère biocompatible.

A cet effet, la tige présente un logement pour le support de capture, ledit logement étant agencé de sorte que la surface du support de capture soit en retrait de la surface circonférentielle de la tige.

De manière particulièrement avantageuse, le support de capture comprend du silicium ou un matériau organosilicié nanoporeux.

Selon un mode de réalisation, la région distale de la tige comprenant le support de capture est sécable.

La gaine peut avantageusement être en polytétrafluoroéthylène.

Selon une forme d'exécution, l'extrémité proximale de la tige est couplée à un moyen d'actionnement.

### BREVE DESCRIPTION DES DESSINS

D'autres caractéristiques et avantages de l'invention ressortiront de la description détaillée qui va suivre, en référence aux dessins annexés sur lesquels :
- la figure 1 est une vue de côté d'un dispositif de prélèvement conforme à l'invention, dans sa configuration rétractée,
- la figure 2 est une vue de côté d'un dispositif de prélèvement conforme à l'invention, dans sa configuration déployée,
- la figure 3 présente des spectres d'analyse MALDI d'un échantillon de périlymphe sur une surface inox conventionnelle (a) et sur la surface d'un support de capture utilisé dans l'invention (b).

### DESCRIPTION DETAILLEE DE L'INVENTION

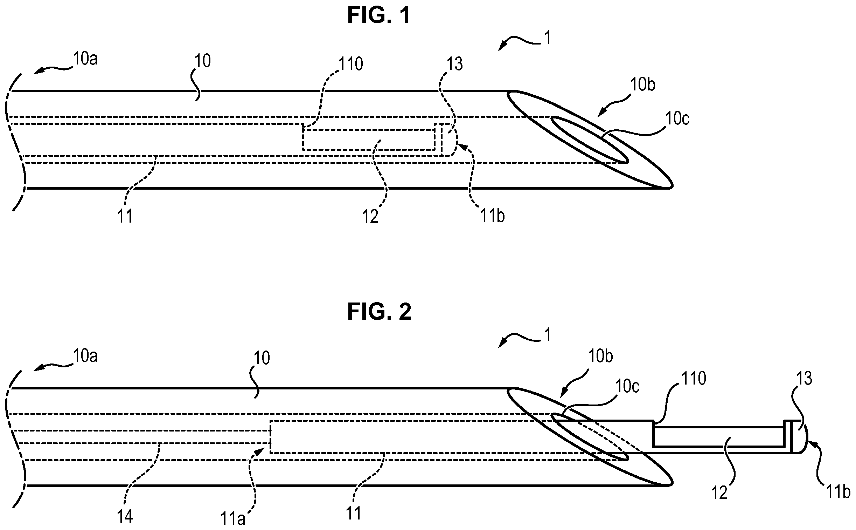

La figure 1 illustre un dispositif de prélèvement in vivo d'espèces biologiques dans sa configuration rétractée. La figure 2 illustre ledit dispositif dans sa configuration déployée.

Le dispositif 1 comprend une gaine tubulaire 10 s'étendant entre une extrémité proximale 10a et une extrémité distale 10b.

L'extrémité distale 10b présente une partie saillante s'étendant parallèlement à l'axe longitudinal de la gaine, adaptée pour perforer la membrane entourant l'organe dans lequel le prélèvement doit être effectué.

Par exemple, ladite extrémité distale présente une forme biseautée. L'angle du biseau est suffisamment prononcé pour permettre une incision de la membrane sans qu'une pression d'appui élevée ne soit appliquée sur l'organe. Ce biseau peut être réalisé par un usinage de l'extrémité distale de la gaine tubulaire.

De manière avantageuse, ladite extrémité distale est tranchante, afin de faciliter l'incision et minimiser l'endommagement des tissus traversés.

La gaine tubulaire 10 présente un canal intérieur cylindrique débouchant à l'extrémité distale 10b par une ouverture 10c.

Le dispositif 1 comprend par ailleurs une tige 11 s'étendant entre une extrémité proximale 11a et une extrémité distale 11b.

La tige 11 est apte à coulisser dans le canal intérieur de la gaine 10 entre une position rétractée dans laquelle l'extrémité distale 11b de la tige est située à l'intérieur de la gaine 10 (cf. figure 1) et une position déployée dans laquelle l'extrémité distale 11b de la tige 11 s'étend au-delà de l'extrémité distale 10b de la gaine, au travers de l'ouverture 10c (cf. figure 2).

La tige 11 comprend un support 12 de capture desdites espèces biologiques, qui est réalisé en un matériau poreux.

De manière avantageuse, la tige 11 est réalisée en un matériau biocompatible, tel que du polyétheréthercétone (PEEK).

De manière avantageuse, le support 12 est réalisé en silicium ou en un matériau organosilicié nanoporeux et rendu solidaire de la tige 11 au moyen d'une colle biocompatible ou de tout autre moyen de fixation approprié.

A cet effet, la tige présente avantageusement dans une portion de sa circonférence un logement 110 dont les dimensions sont adaptées pour recevoir le support 12. Le support 12 est avantageusement agencé en retrait dans le logement 110 par rapport à la surface circonférentielle de la tige, de sorte que ses bords, qui peuvent être coupants, ne viennent pas agresser l'organe dans lequel le prélèvement est effectué. D'autre part, cet agencement évite également tout frottement entre le support de capture 12 et la paroi intérieure de la gaine 10 pendant la manipulation de la tige 11.

Le support de capture 12 est agencé dans une région distale de la tige 11, de telle sorte que ledit support 12 soit situé à l'extérieur de la gaine 10 lorsque la tige 11 est dans sa position déployée (cf. figure 2).

De manière avantageuse, la partie distale de la tige comprenant le support de capture est sécable, ce qui permet de disposer facilement du support de capture en vue de l'analyse des espèces prélevées.

A son extrémité proximale 11a, la tige 11 est couplée à un moyen d'actionnement 14. Ledit moyen s'étend dans la direction proximale au-delà de l'extrémité proximale de la gaine 10 et est destiné à être manipulé par le praticien pour faire coulisser la tige entre la position rétractée et la position déployée. Ledit moyen d'actionnement 14 comprend avantageusement un câble ou un tube flexible, ayant un diamètre plus faible que celui de la tige 11 et présentant une rigidité suffisante pour exercer une poussée de la tige.

A son extrémité distale 11b, la tige 11 présente de préférence un embout 13 arrondi de manière à ne pas provoquer de lésion de l'organe dans lequel on effectue le prélèvement. Ledit embout 13 peut faire partie intégrante de la tige et être ainsi formé du même matériau que ladite tige, ou bien être réalisé en un autre matériau puis rendu solidaire de la tige par tout moyen approprié.

La gaine est avantageusement réalisée en polytétrafluoroéthylène (PTFE) ou en un autre matériau présentant un faible coefficient de frottement.

Ainsi, la gaine protège la tige et le support de capture situé dans la gaine avant et après le prélèvement proprement dit, tout en facilitant le coulissement de la tige entre sa position rétractée et sa position déployée.

Le support de capture n'est exposé aux tissus ou aux fluides corporel du patient que lorsque l'extrémité distale de la gaine 10 a été placée à l'endroit adéquat dans l'organe dans lequel le prélèvement doit être effectué.

A titre purement indicatif, la gaine 10 présente une longueur d'au moins 10 cm, un diamètre extérieur compris entre 1 et 2 mm et un diamètre intérieur compris entre 0,7 et 1,3 mm.

La tige 11 présente un diamètre compris entre 0,3 et 0,7 mm et une longueur d'au moins 2 cm.

Comme indiqué plus haut, le support de capture 12 présente de préférence une forme inscrite dans le diamètre de la tige 11. La longueur du support 12 peut varier en fonction des conditions du prélèvement prévu et est typiquement comprise entre 1 mm et 1 cm.

Le support de capture est avantageusement réalisé en silicium nanoporeux, l'expérience ayant montré que ce matériau convient bien à la capture de petites protéines, c'est-à-dire dont le ratio m/z (où m désigne la masse de la molécule et z la charge) est inférieur à 8000 voire à 1000.

La nanoporosité de la surface du support de capture peut être obtenue de différentes manières. Selon la méthode employée, l'épaisseur poreuse, la densité de porosité et la taille des pores peut varier. La variation de ces caractéristiques modifie la nature des molécules (selon la gamme de masse) capturées et analysables en spectrométrie de masse MALDI. Typiquement, l'épaisseur poreuse est supérieure à 100 nm, la densité de pores est comprise entre 10 et 75% et la taille des pores est comprise entre 1 et 100 nm.

Cette surface nanoporeuse peut résulter d'une attaque électrochimique d'un substrat de silicium. L'épaisseur sur laquelle le substrat est rendu poreux est typiquement supérieure à 100 nm et peut être toute l'épaisseur du substrat. Par exemple, on peut obtenir par attaque électrochimique une épaisseur poreuse peut être de l'ordre de 2,2 µm, une densité de porosité de 40% et une taille des pores comprise entre 10 et 15 nm.

De manière alternative, la surface nanoporeuse peut résulter du dépôt d'une couche d'un matériau organosilicié poreux, de type SiOCH, sur un substrat. Par exemple, une couche de SiOCH est déposée par PECVD (acronyme du terme anglo-saxon « Plasma Enhanced Chemical vapor Deposition » par co-dépôt d'une matrice d'organosiliciés et de composés organiques thermiquement labiles (agents porogènes). Les agents porogènes sont ensuite évacués par un recuit UV à 400°C pendant quelques minutes. La couche de SiOCH ainsi obtenue présente une épaisseur contrôlée pouvant être comprise entre 180 nm et 1000 nm, une porosité ouverte et interconnectée maximale de 30% (mesure à l'ellipsoporosimétrie en utilisant du Toluène), un diamètre moyen des pores de 1,3 nm et est hydrophobe avec un angle de contact de l'ordre de 100°. Les caractéristiques physico-chimiques de la couche peuvent ensuite être modifiées par un plasma de post-traitement. Ainsi, un plasma N2H2 permet d'obtenir une porosité de 35% et un angle de contact de 80°.

De manière alternative, le support de capture peut être en métal rendu nanoporeux par attaque électrochimique (par exemple à base d'acide fluorhydrique), ou comprendre une couche de polymère organique poreux (par exemple copolymère dibloc) déposée sur un substrat.

Le protocole d'introduction du dispositif 1 pour le prélèvement de périlymphe dans la cochlée est similaire à celui de la mise en place d'un implant cochléaire.

Le dispositif est introduit de la façon suivante : l'approche jusqu'à la cochlée est réalisée par voie d'abord rétroauriculaire avec réalisation d'une masto-antroatticotomie puis réalisation d'une tympanotomie postérieure. La membrane de la fenêtre ronde (« round window membrane » selon la terminologie anglo-saxonne) est alors exposée. On peut alors introduire le dispositif. Pendant cette étape d'introduction, le dispositif est dans sa position rétractée, le support de capture n'étant donc pas exposé aux tissus et fluides biologiques.

Le geste du praticien est guidé optiquement (binoculaire), de sorte que le praticien visualise la position de la partie saillante de l'extrémité distale de la gaine par rapport à la membrane.

Une fois que la membrane est percée, le praticien cesse l'introduction de la gaine et déploie la tige de manière à mettre le support de capture en contact avec la périlymphe. On prélève ainsi sur le support de capture des espèces biologiques susceptibles d'être impliquées dans les dysfonctionnements de l'audition, telles que des protéines (notamment la lipofuscine).

Lors de cette opération, l'embout 13 de la tige peut éventuellement venir s'appuyer sur la coque osseuse à l'intérieur de la cochlée. Le fait que cet embout soit arrondi permet d'éviter de provoquer une lésion de la cochlée.

Puis la tige est à nouveau rentrée dans la gaine tubulaire et le dispositif rétracté est retiré du corps du patient.

L'exemple décrit plus haut n'est qu'une illustration particulière qui n'est pas limitative quant aux domaines d'application de l'invention. Ainsi, outre le prélèvement de périlymphe dans la cochlée, le dispositif décrit plus haut peut également être utilisé pour des prélèvements de liquides biologiques dans des glandes telles que les glandes salivaires, les glandes lacrymales ou encore les sinus.

### Exemple expérimental

Pour valider la capacité du support de capture en silicium nanoporeux à prélever des espèces biologiques, le protocole décrit ci-après a été mis en oeuvre à partir d'un prélèvement de périlymphe au cours d'une opération sur un rat.

Le volume du prélèvement était d'une dizaine de microlitres.

Deux puces de silicium de 5 mm par 5 mm avec des pores de 1 à 2 nm (couche de SiOCH déposée par PECVD comme décrit plus haut), nettoyées par ultra-sonification dans de l'acétone pendant 3 min, ont été préparées.

La 9AA (9-aminoacridine) adaptée à l'analyse MALDI de métabolites et peptides (jusqu'à 1000 daltons) a été utilisée sur la surface de SiOCH qui, de par la dimension de ses pores, enrichit essentiellement ce type de molécule.

1 µL de périlymphe a été déposé sur l'une des deux puces de silicium, l'autre étant laissée nue en contrôle.

De la 9AA (9-aminoacridine - matrice MALDI) a été déposée sur les deux puces, ceci afin d'étudier le métabolome de la périlymphe.

La périlymphe a été incubée 5 minutes sur le support poreux avant rinçage par une solution aqueuse TFA 0,1% et analysée en spectrométrie de masse MALDI.

Afin de valider l'intérêt du SiOCH nanoporeux, le même échantillon de périlymphe a été analysé de manière traditionnelle sur une plaque MALDI présentant une surface d'acier inox non poreuse.

Paramètres de lecture sur le spectromètre de masse MALDI (Brucker Ultraflex) : l'acquisition automatique des spectres est réalisée sur 5000 impacts laser en mode réflectron positif, une intensité laser à 75% et une atténuation du signal de matrice à 200 daltons et à 0 daltons. Autrement dit, les molécules dont le rapport m/z est inférieur à 200 Daltons, en majorité issues de la matrice 9-aminoacridine, sont déviées afin d'éviter la saturation du détecteur.

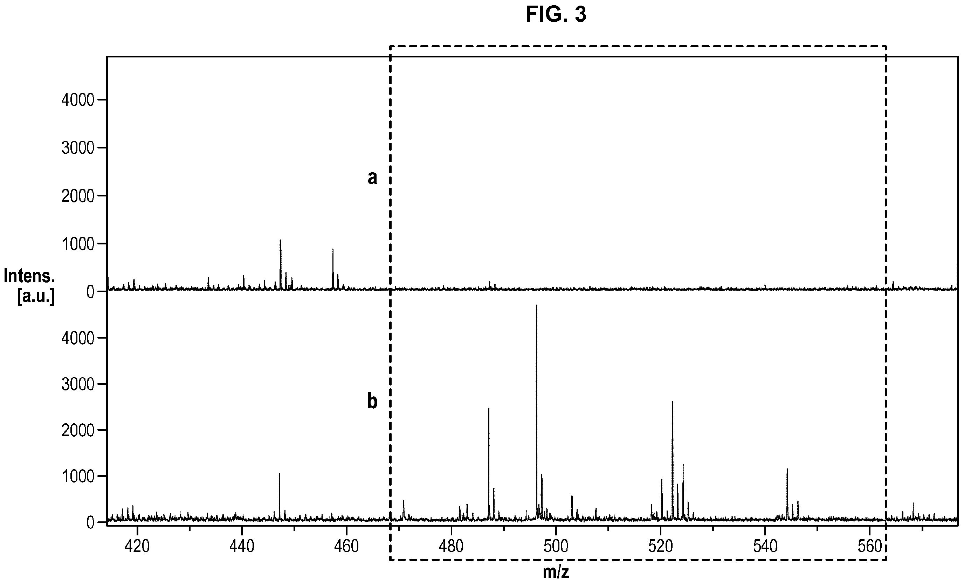

La figure 3 présente dans la partie haute (a) le spectre de la périlymphe sur la surface inox et dans la partie basse (b) le spectre de la périlymphe sur la surface du silicium poreux. L'axe des abscisses est gradué selon une échelle de masse (m/z) ; l'axe des ordonnées correspond à une échelle arbitraire identique pour les deux spectres.

On observe dans la zone entourée un enrichissement visible du spectre protéique, peptidique et métabolomique sur la surface de silicium poreux par rapport à la surface inox.

### REFERENCES

[1] Swan EE, Peppi M, Chen Z, Green KM, Evans JE, McKenna MJ, Mescher MJ, Kujawa SG, Sewell WF. Proteomics analysis of perilymph and cerebrospinal fluid in mouse. Laryngoscope. 2009 May; 119(5):953-8
[2] Lysaght AC, Kao SY, Paulo JA, Merchant SN, Steen H, Stankovic KM. Proteome of human perilymph. J Proteome Res. 2011 Sep 2; 10(9):3845-51
[3] Salt AN, Kellner C, Hale S. Contamination of perilymph sampled from the basal cochlear turn with cerebrospinal fluid. Hear Res 2003; 182:24-33
[4] Salt AN, Hale SA, Plontke SK. Perilymph sampling from the cochlear apex: a reliable method to obtain higher purity perilymph samples from the scala tympani. J Neurosci Meth 2006; 153:121-129

## Revendications

1. Dispositif (1) de prélèvement in vivo d'espèces biologiques, comprenant :
- une gaine (10) tubulaire s'étendant entre une extrémité proximale (10a) de ladite gaine et une extrémité distale (10b) de ladite gaine, ladite extrémité distale (10b) de la gaine présentant une partie saillante adaptée pour perforer une membrane d'un organe contenant les espèces biologiques à prélever,
- une tige (11) s'étendant entre une extrémité proximale (11a) de ladite tige et une extrémité distale (11b) de ladite tige, apte à coulisser dans la gaine (10) entre une position rétractée dans laquelle l'extrémité distale (11b) de la tige est située à l'intérieur de la gaine (10) et une position déployée dans laquelle l'extrémité distale (11b) de la tige s'étend au-delà de l'extrémité distale (10b) de la gaine,
ladite tige (11) comprenant un support (12) de capture desdites espèces biologiques, en un matériau poreux, agencé dans une région distale de la tige sur une portion de la circonférence de la tige (11) de telle sorte que le support de capture (12) soit situé à l'extérieur de la gaine (10) lorsque la tige (11) est dans sa position déployée, **caractérisé en ce que** ledit support de capture (12) est agencé dans un logement de la tige (110) configuré pour que la surface du support de capture (12) soit en retrait de la surface circonférentielle de la tige.

2. Dispositif selon la revendication 1, dans lequel l'extrémité distale (10b) de la gaine tubulaire forme un biseau.

3. Dispositif selon l'une des revendications 1 ou 2, dans lequel l'extrémité distale de la tige comprend un embout (13) arrondi.

4. Dispositif selon la revendication 3, dans lequel ledit embout (13) est réalisé en un polymère biocompatible.

5. Dispositif selon l'une des revendications 1 à 4, dans lequel le support de capture (12) comprend du silicium ou un matériau organosilicié nanoporeux.

6. Dispositif selon l'une des revendications 1 à 5, dans lequel la région distale de la tige comprenant le support de capture (12) est sécable.

7. Dispositif selon l'une des revendications 1 à 6, dans lequel la gaine (10) est en polytétrafluoroéthylène.

8. Dispositif selon l'une des revendications 1 à 7, dans lequel l'extrémité proximale (11a) de la tige (11) est couplée à un moyen d'actionnement (14).

## Patentansprüche

1. in vivo-Entnahmevorrichtung (1) biologischer Arten, umfassend:
- einen röhrenförmigen Kanal (10), der sich zwischen einem proximalen Ende (10a) des genannten Kanals und einem distalen Ende (10b) des genannten Kanals erstreckt, wobei das genannte distale Ende (10b) des Kanals einen hervorstehenden Teil aufweist, der geeignet ist, um eine Membran eines Organs zu perforieren, das die zu entnehmenden biologischen Arten enthält;
- einen Stift (11), der sich zwischen einem proximalen Ende (11a) des genannten Stiftes und einem distalen Ende (11b) des genannten Stiftes erstreckt und geeignet ist, in dem Kanal (10) zwischen einer eingezogenen Position, in der das genannte distale Ende (11b) des Stiftes im Innern des Kanals (10) angeordnet ist, und einer ausgefahrenen Position, in der das genannte distale Ende (11b) des Stiftes sich über das distale Ende (10b) des Kanals hinaus erstreckt, zu gleiten,
wobei der genannte Stift (11) einen Träger (12) zur Erfassung der genannten biologischen Arten aus einem porösen Material, das in einer distalen Region des Stiftes auf einem Abschnitt des Umfangs des Stiftes (11) derart angeordnet ist, dass der Träger zur Erfassung (12) an der Außenseite des Kanals (10) angeordnet ist, wenn der Stift (11) in seiner ausgefahrenen Position ist, umfasst, **dadurch gekennzeichnet, dass** der genannte Träger zur Erfassung (12) in einer Aufnahme des Stiftes (110) angeordnet ist, die konfiguriert ist, damit die Fläche des Trägers zur Erfassung (12) von der Umfangsfläche des Stiftes zurückgesetzt ist.

2. Vorrichtung gemäß Anspruch 1, bei dem das distale Ende (10b) des röhrenförmigen Kanals eine Fase bildet.

3. Vorrichtung gemäß einem der Ansprüche 1 oder 2, bei der das distale Ende des Stiftes eine abgerundete Endabdeckung (13) umfasst.

4. Vorrichtung gemäß Anspruch 3, bei der die genannte Endabdeckung (13) aus biokompatiblem Polymer realisiert ist.

5. Vorrichtung gemäß einem der Ansprüche 1 bis 4, bei der der Träger zur Erfassung (12) Silizium oder ein organisch-siliziertes, nanoporöses Material umfasst.

6. Vorrichtung gemäß einem der Ansprüche 1 bis 5, bei der die distale Region des Stiftes, die den Träger zur Erfassung (12) umfasst, abtrennbar ist.

7. Vorrichtung gemäß einem der Ansprüche 1 bis 6, bei der der Kanal (10) aus Polytetrafluorethylen ist.

8. Vorrichtung gemäß einem der Ansprüche 1 bis 7, bei der das proximale Ende (11a) des Stiftes (11) an ein Betätigungsmittel (14) gekoppelt ist.

## Claims

1. Device (1) for *in vivo* sampling of biological species, comprising:
- a tubular sheath (10) extending between a proximal end (10a) of said sheath and a distal end (10b) of said sheath, said distal end (10b) of the sheath having a projecting part adapted to perforate a membrane of an organ containing the biological species to sample.
- a rod (11) extending between a proximal end (11a) of said rod and a distal end (11b) of said rod, capable of sliding in the sheath (10) between a retracted position in which the distal end (11b) of the rod is located inside the sheath (10) and a deployed position in which the distal end (11b) of the rod extends beyond the distal end (10b) of the sheath,
said rod (11) comprising a capturing support (12) for capturing said biological species, made from a porous material, arranged in a distal region of the rod on a portion of the circumference of the rod (11) such that the capturing support (12) is located outside the sheath (10) when the rod (11) is in the deployed position of same, **characterised in that** said capturing support (12) is arranged in a housing of the rod (110) configured such that the surface of the capturing support (12) is set back from the circumferential surface of the rod.

2. Device according to claim 1, in which the distal end (10b) of the tubular sheath forms a bevel.

3. Device according to one of claims 1 or 2, in which the distal end of the rod comprises a rounded tip (13) .

4. Device according to claim 3, in which said tip (13) is made from a biocompatible polymer.

5. Device according to one of claims 1 to 4, in which the capturing support (12) comprises nanoporous silicon or an organosilicon material.

6. Device according to one of claims 1 to 5, in which the distal region of the rod comprising the capturing support (12) is breakable.

7. Device according to one of claims 1 to 6, in which the sheath (10) is made from polytetrafluoroethylene

8. Device according to one of claims 1 to 7, in which the proximal end (11a) of the rod (11) is coupled to an actuating means (14).
